(19) Europäisches Patentamt
European Patent Office
Office européen des brevets

(11) Publication number: **0 137 640**
**A1**

(12) # EUROPEAN PATENT APPLICATION

(21) Application number: 84305510.4

(22) Date of filing: 13.08.84

(51) Int. Cl.⁴: C 07 D 475/08
//A61K31/505

(30) Priority: 15.08.83 US 523269

(43) Date of publication of application:
17.04.85 Bulletin 85/16

(84) Designated Contracting States:
CH DE FR GB IT LI SE

(71) Applicant: DANA-FARBER CANCER INSTITUTE, INC.
44 Binney Street
Boston Massachusetts 02115(US)

(72) Inventor: Rosowsky, Andre
76 Lindbergh Avenue
Needham Massachusetts(US)

(74) Representative: Marsh, Roy David et al,
Brewer & Son 5-9, Quality Court Chancery Lane
London WC2A 1HT(GB)

(54) **Chain extended analogues of methotrexate and aminopterin.**

(57) Chain extended analogues of Methotrexate and Aminopterin having antitumor activity and low toxicity are soluble in water at a physiological pH ranging from 7.2-7.5.

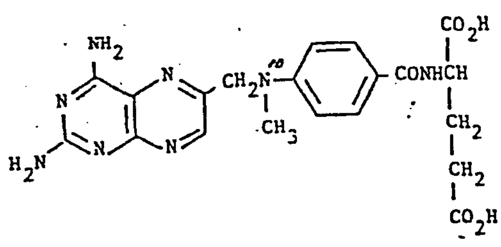

FIG. 1

EP 0 137 640 A1

Croydon Printing Company Ltd.

CHAIN EXTENDED ANALOGUES OF METHOTREXATE AND AMINOPTERIN

The invention relates to certain novel chemical compounds having antitumor activity against L1210 leukemias in mice together with low toxicity and to therapeutic compositions containing these compounds or certain related compounds, together with a pharmaceutically acceptable non-toxic carrier, which are useful for administration to mice and other mammals having certain tumors for extending their life spans.

Methotrexate (MTX; 4-amino-4-deoxy-$N^{10}$-methylpteroylglutamic acid) and Aminopterin (AMT; 4-amino-4-deoxy-pteroylglutamic acid) are folate antagonists and act as antineoplastic agents by interfering with one or more biosynthetic steps involving folate coenzymes of the tumor cell. The structure of MTX differs from AMT in that the former contains a methyl group in the $N^{10}$ position while the latter does not, having hydrogen instead. The structural formula of MTX is as follows:

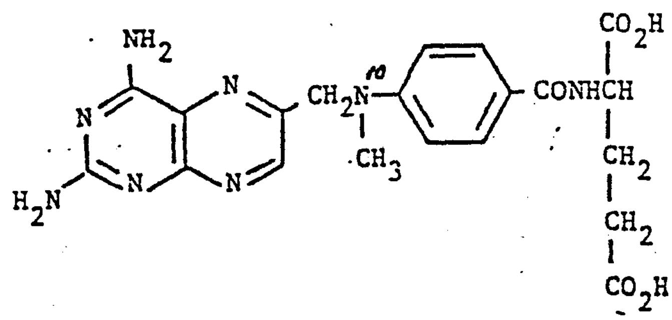

MTX and AMT have been found to be effective clinically against certain malignant tumors: for example, good to excellent tumor response has been seen in patients with acute lymphocytic leukemia, Burkitt's

lymphoma, carcinoma of the breast, mycosis fungoides, epidermoid cancer of the head and neck area, and osteogenic sarcoma. In addition, MTX is the drug of choice in the treatment of choriocarcinoma and is also used for certain non-neoplastic conditions such as generalized psoriasis and certain autoimmune diseases such as rheumatoid arthritis and lupus erythematosus. However, chemotherapy with MTX or AMT is accomplished by a variety of toxicities, particularly their ability to form polyglutamates, which limit the effectiveness of the compounds and their long-term use.

The novel compounds of the present invention comprise MTX and AMT analogues in which the dicarboxylic acid portion of the molecule contains from 6-25, and preferably from 6-15, carbon atoms. These compounds have the following generic structure:

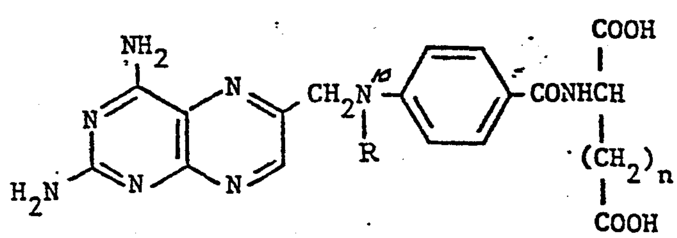

in which R=$CH_3$ or H; n=3-22, preferably 3-12. They are compounds in which the glutamic acid moiety of MTX or AMT is replaced by an L-α-amino dicarboxylic acid having 6-25 carbon atoms. Among such L-α-amino dicarboxylic acids are: L-α-aminoadipic, L-α-aminopimelic, L-α-aminosuberic, L-α-aminoazelaic, L-α-aminosebacic, L-α-aminoundecanedioic, L-α-amino-dodecanedioic, and L-α-aminotetradecanedioic.

The novel compounds of the present invention are prepared by the condensation of $N^{10}$-protected-4-amino-4-deoxy-pteroic acid or $N^{10}$-protected-4-amino-4-deoxy-$N^{10}$-methylpteroic acid with a diester of an L-α-amino dicarboxylic acid containing 6-25 carbon atoms, followed by hydrolysis with base. Coupling is accomplished in up to 85% yield by the use of the peptide-bond-forming reagent diethyl phosphorocyanidate at room temperature. For example, the compounds of examples 1-3 are synthesized by the condensation of 4-amino-4-deoxy-$N^{10}$-methylpteroic acid with the diesters of L- α-aminoadipic, L- α-aminopimelic, and L- α-aminosuberic acids.

Therapeutic compositions containing the novel compounds of the present invention as the active agents can be prepared by dispersing or dissolving the active agent in any pharmaceutically acceptable non-toxic carrier suitable for the desired mode of administration, which may be parenteral, that is, by injection or infusion which is intravenous, intracavitary, or other conventional mode. Preferably the carrier is an aqueous medium buffered to pH 7.2-7.5, the physiological range. Any suitable conventional buffer can be used such as phosphate, bicarbonate, or citrate. If desired, saline solution can be used, with pH adjustment and buffering. Dosages may vary over a wide range depending upon individual conditions and can readily be determined using the dosages commonly employed for MTX or AMT as exemplars.

The toxicity and therapeutic effectiveness of the compounds of the present invention are shown by in vitro assays and by in vivo evaluations in mice. The cytotoxicity of the compounds against L1210 cells in

culture is measured according to the method described by Rosowsky et al. (1982) J. Med. Chem., 25, 171. The results show that cytotoxicity to L1210 cells in culture is comparable to, or slightly higher than that of MTX or AMT, indicating that the ability of the compounds of the present invention to cross the cell membrane is preserved. Furthermore, cytotoxicity increases as the number of $CH_2$ groups in the amino acid side chain is extended.

The ability of the compounds of the present invention to act as substrates or inhibitors for the enzymes dihydrofolate reductase, folate polyglutamate synthetase, and carboxypeptidase G1 is also measured in in vitro assays.

Dihydrofolate reductase assays are performed as described by Rosowsky et al. (1981) J. Med. Chem. 24, 1450. The results of the dihydrofolate reductase assays show that the compounds of the present invention bind to bacterial (Lactobacillus casei) and mammalian (L1210 mouse leukemia) dihydrofolate reductase with an affinity comparable to MTX and AMT and that increasing the distance between the side-chain groups is not detrimental to binding.

Folate polyglutamate synthetase assays are performed as described by Forsch et al. (1983) AACR Proc. 24. The results of the folate polyglutamate synthetase assays show that, unlike MTX and AMT, the compounds of the present invention are poor substrates for the enzyme and therefore are not expected to form polyglutamates intracellularly. This property, which presumably accounts for the higher dosage and frequency requirements of these compounds relative to MTX or AMT, is of particular interest and is a therapeutic asset.

In patients with MTX or AMT resistant tumors, where resistance is associated with a lower capacity for polyglutamation than for normal proliferative tissues, dose escalation will be tolerated better with the compounds of the present invention than with MTX or AMT, since the compounds of this invention are not polyglutamated and thus have a faster clearance rate and lower toxicity than MTX or AMT. Thus, the compounds of the present invention whose cytotoxic action does not involve polyglutamation will offer an advantage, since a tumor with a low capacity for polyglutamate formation would be no less sensitive than normal proliferative tissues. Additionally, it should be noted that the compounds of the present invention may find use in longterm low-dose regimens, e.g., in psoriasis or rheumatoid arthritis treatment, where progressive MTX or AMT polyglutamate accumulation in hepatocytes may be responsible for the chronic hepatotoxicity typically associated with this type of therapy.

Carboxypeptidase G1 assays are performed as described by McCullough et al. (1971) J. Biol. Chem. 246, 7207. The results of the carboxypeptidase G1 assays show that while MTX is rapidly cleaved by the enzyme, as described by Chabner et al. (1972) Nature, 239, 395, the compounds of the present invention are not. This property is of particular interest and is a therapeutic advantage since carboxypeptidase G1 can be used to create a deficiency in reduced folates and thus potentiate the therapeutic effect of the compounds of the present invention without inactivating them.

In vivo antitumor activity is determined against L1210 ascitic leukemia in mice according to a standard NCI protocol, Geran et al. (1972) Cancer Chemother. Rep., 3(3), 1. The results of the in vivo

antitumor assays against L1210 leukemia in mice show that the compounds of the present invention increase survival to about the same extent as MTX or AMT.

The following examples are intended to illustrate more fully the preparation of the compounds of the present invention without acting as a limitation upon the scope of the invention.

Example 1

N-(4-amino-4-deoxy-$N^{10}$-methylpteroyl)-L-α-aminoadipic acid.

L-α-aminoadipic acid is synthesized from N-(benzyloxycarbanoyl)-L-lysine according to the method of Scott and Wilkinson (1980) Synth. Commun. 10, 127. Anhydrous HCl gas is bubbled through a suspension of L-α-aminoadipic acid (4.0 g, 0.025 mol) in absolute EtOH (25 ml). Another 50 ml of EtOH is added, the mixture is stirred under reflux overnight, and the solvent is evaporated. The residue is partitioned between $Et_2O$ and aqueous $K_2CO_3$, and the organic layer is washed with brine, dried, and evaporated to a small volume. Addition of ethereal HCl gives the hydrochloride salt of diethyl l-α-aminoadipate (2.7 g, 42%) as a waxy solid.

The analytical sample is recrystallized from EtOAc and dried in vacuo at 60 °C over $P_2O_5$ overnight: mp 107-109 °C; IR (KBr) ν 3410, 2930, 1735 (ester C=O) $cm^{-1}$. Analysis for C, H, N, and Cl confirms the composition.

Coupling of the amino ester to 4-amino-4-deoxy-$N^{10}$-methylpteroic acid

4-Amino-4-deoxy-$N^{10}$-methylpteroic acid (1.6 g, 0.005 mol) is added in small portions to a stirred solution of diethyl phosphorocyanidate (2.5 g, 0.015 mol) and $Et_3N$ (1.5 g, 0.015 mol) in dry N,N-dimethylformamide (DMF) (150 ml). The solution is heated in an oil bath at 80 °C for a few minutes, then cooled, and treated with another portion of $Et_3N$ (1.0 g, 0.010 mol) followed by diethyl L-α-aminoadipate·HCl (1.3 g, 0.005 mol). The reaction mixture is heated again at 80 °C for 2 h, cooled, and evaporated to dryness under reduced pressure. The residue is taken up in $CHCl_3$, and the solution is washed with $H_2O$ and evaporated. Column chromatograhy on silica gel (95:5 $CHCl_3$-MeOH), pooling of appropriate column eluates, and trituration of the evaporated fractions with $Et_2O$ yields the diester, diethyl N-(4-amino-4-deoxy-$N^{10}$-methylpteroyl)-L-α-aminoadipate, as a bright-yellow powder (1.2 g, 51%): mp 113-123 °C; IR (KBr) ν 3390, 2920, 1720 (ester C=O), 1615-1640 (amide C=O) $cm^{-1}$. Analysis for C, H, and N confirms the composition.

A solution of the diester (1 g, 0.002 mol) in a mixture of 95% EtOH (30 ml) and $H_2O$ (25 ml) is stirred at room temperature overnight with $Ba(OH)_2 \cdot 8H_2O$ (1.26 g, 0.004 mol). To the suspension is then added a solution of $Na_2SO_4$ (0.57 g, 0.004 mol) in a minimum of $H_2O$. After a few minutes of stirring, the $BaSO_4$ is filtered off,and the aqueous layer is extracted with $CHCl_3$ and acidified with 10% AcOH. The precipitate is

filtered and dried under high vacuum in a lyophilization apparatus to obtain N-(4-amino-4-deoxy-$N^{10}$-methyl-pteroyl)-L-α-aminoadipic acid as a yellow powder (0.8 g, 85%). This material is pooled with the product (1.2 g, 78%) from another larger run, of the same procedure, and purified by ion-exchange chromatography on DEAE-cellulose, using 3% ammonium bicarbonate as the eluent. Appropriate TLC-pure fractions are combined and reduced to a small volume by lyophilization, and the product is precipitated by the addition of 10% AcOH; $R_f$ 0.8 (cellulose, 0.1 M phosphate buffer, pH 7.4); IR (KBr) ν 3360, 1610-1640 $cm^{-1}$. Analysis for C, H, and N confirms the composition of the compound of example 1.

Example 2

N-(4-amino-4-deoxy-$N^{10}$-methylpteroyl)-L-α-aminopimelic acid.

L-α-aminopimelic acid is synthesized as follows. N-(tert-butyloxycarbonyl)-L-glutamic acid α-benzyl ester (10.1 g, 0.03 mol) is condensed with 2,2-dimethyl-1,3-dioxane-4,-dione ("Meldrum's acid") (3.60 g, 0.025 mol) in dry DMF (125 ml) using diethyl phosphorocyanidate (4.90 g, 0.03 mol) and $Et_3N$ (8.05 g, 0.08 mol) as the coupling reagent in a modification of the method of Shioiri and Hamada (1978) J. Org. Chem. 43, 3631. The mixture is stirred in the ice-bath for 2 h, and then left at room temperature overnight. Rotary evaporation left an oil, which is taken up in 1:1 benzene-EtOAc. After the solution is washed with $H_2O$, 5% $NaHCO_3$, and brine, the solvents are evaporated under reduced pressure to obtain benzyl 2-[N-(tert-butyloxycarbonyl)amino]-5-[5-(2,2-dimethyl-1,3-dioxane-4,6-dionyl)]pentanoate as a gum. A solution of this

material in glacial AcOH (35 ml) is treated with $NaBH_3CN$ (3.14 g, 0.05 mol) over 1-2 min, and after being stirred for 1 h the mixture is diluted with ice-cold 0.5 N HCl (125 mL) and extracted with benzene. The organic layer is washed successively with $H_2O$, 5% $NaHCO_3$, and brine to obtain the crystalline dilactone as a white solid (8.46 g, 75%). The analytical sample is prepared by column chromatography on silica gel (95:5 $CHCl_3$-MeOH): mp 110-112 °C (slow gas evolution at 120 °C); IR (KBr) ν 3350, 2940, 1775sh, 1745, 1680, 1510 $cm^{-1}$; NMR ($CDCl_3$) δ 1.4-2.2 [m, 21H, t-$C_4H_9$, $(CH_2)_3$, and $C(CH_3)_2$], 3.51 (m, 1H, malonyl CH), 4.0-4.5 (m, 2H, α-CH and NH), 5.19 (s, 2H, benzylic $CH_2$), 7.35 (s, 5H, aromatic protons). Analysis for C, H, and N confirms the composition. (N: Calcd, 3.03; Found, 3.53.) It should be noted that the novel application of Meldrum's acid described here can be used in the synthesis of other amino dicarboxylic acids of longer chain length, for use in making other compounds of the present invention.

The dilactone (2.3 g, 0.005 mol) is heated overnight in refluxing 6 N HCl (15 ml), and the cooled solution is extracted with $Et_2O$. The aqueous layer is evaporated under reduced pressure, and the crude HCl with pyridine. Absolute EtOH (15 ml) is added, and the mixture is left in the freezer overnight. The crystals are filtered, washed with 95% EtOH, and dried in vacuo over $P_2O_5$ at 95 °C to obtain analytically pure L-α-aminopimelic acid (0.66 g, 75%): $D^{25}$ +21.6° (c = 1, 5 M HCl) [Lit.[15] +21.6°; lit.[23] +21.5°]. Analysis for C, H, and N confirms the composition of L-α-amino pimelic acid.

A mixture of L-α-aminopimelic acid (0.35 g, 0.0002 mol) and 70% $HClO_4$ (0.32 g, 0.0022 mol) in *tert*-butyl acetate (30 ml) is stirred at room temperature overnight until it becomes homogenous. After an additional day, the solution is extracted with cold 0.5 n HCl (2 x 10 ml), the aqueous layer is carefully neutralized with small portions of powdered $NaHCO_3$, and the product is extracted into $Et_2O$. The $Et_2O$ layer is washed with brine, dried, and evaporated to obtain the diester as an oil (0.31 g, 54%); IR (neat)ν 3400sh 2980, 1730 (C=O) $cm^{-1}$.

4-Amino-4-deoxy-$N^{10}$-methylpteroic acid (0.37 g, 0.001 mol) is added in small portions to a solution of diethyl phosphorocyanidate (0.51 g, 0.0031 mol) and $Et_3N$ (0.3 g, 0.003 mol) in dry DMF (35 ml). After 2 min in an oil bath at 80 °C, the solution is cooled, and another portion of $Et_3N$ (0.1g, 0.001 mol) is added followed by a solution of di-*tert*-butyl L-α -aminopimelate (0.31 g, 0.001 mol) in a small volume of DMF. The reaction mixture is heated at 80 °C for 2 h, and then left overnight at room temperature. Rotary evaporation gives a semisolid that dissolves in $CHCl_3$. The $CHCl_3$ solution is washed with dilute ammonia and evaporated, and the residue is chromatographed on silica gel (95:5 $CHCl_3$-MeOH). Appropriate TLC-homogeneous fractions are pooled and evaporated, and the residue is triturated with $Et_2O$ to obtain di-*tert*-butyl N-(4-amino-4-deoxy-$N^{10}$-methylpteroyl)-L-α-aminopimelate as a bright-yellow powder (0.44 g, 71%): mp 148-150 °C; IR (KBr)ν 3390, 2980, 1725 (ester C=O), 1610-1630 $cm^{-1}$. Analysis for C, H, and N confirms the composition.

The diester (1.8 g, 0.003 mol) is dissolved in trifluoroacetic acid (10 ml), and the solution is left to stand at room temperature for 10 min, before being added dropwise to a stirred solution of $K_2CO_3$ (10 g) in $H_2O$ (40 ml). Additional $K_2CO_3$ is added in small portions until a homogeneous solution is obtained, and the pH is adjusted to 5 with 10% AcOH. After overnight refrigeration, the precipitate is filtered and dried in a high-vacuum lyophilizer to obtain a yellow powder N-(4-amino-4-deoxy-$N^{10}$-methylpteroyl)-L- α -aminopimelic acid 2 (1.4 g, 88% assuming $2.25H_2O$): IR (KBr) ν 3330, 2980sh, 1710sh, 1640, 1600 $cm^{-1}$. Analysis for C, H, and N confirms the composition of the compound of example 2.

Example 3

N-(4-amino-4-deoxy-$N^{10}$-methylpteroyl)-L- α -aminosuberic acid.

A solution of N-(benzyloxycarbonyl)-L- α -aminosuberic acid (0.32 g, 0.001 mol) in dry DMF (5 ml) is treated with cesium carbonate (0.65 g, 0.002 mol) and methyl iodide (0.57 g, 0.004 mol), and the mixture is stirred at room temperature overnight. Rotary evaporation under reduced pressure leaves an oil, which is partitioned between benzene and $H_2O$. After being washed thoroughly with $H_2O$, the benzene layer is evaporated to give the dimethyl ester as an oil: 0.36 g (100%): IR (neat) ν 3340 (NH), 2940, 1725 (broad, C=O) $cm^{-1}$; NMR ($CDCL_3$) δ 1.2-1.9 [m, 8H, $(CH_2)^4$], 2.28 (m. 2H, $CH_2CO_2$-), 3.65 (s, 3H, $OCH_3$), 3.72 (s, 3H, $OCH_3$), 4.05 (1H, α-CH), 5.13 (s. broad m, 3H, NH and benzylic $CH_2$), 7.37 (s, 5H, aromatic protons). The crude N-protected diester (0.35 g, 0.001 mol) in MeOH (20 ml) containing glacial AcOH (0.5 ml) and 5% Pd-C (50 mg) is shaken under $H_2$ 93 atm) for 3 h. The

catalyst is filtered off, the solvent is evaporated, the residue is partitioned between $CH_2Cl_2$ and 5% aqueous $NaHCO_3$, and the organic layer is dried and evaporated to obtain the deblocked diester as an oil: 0.20 g (93%); IR (neat) ν 3280 (NH), 2920, 1725 (ester C=0) $cm^{-1}$; NMR ($CDCL_3$) δ 1.3-1.9 [m, 10H, $(CH_2)_4$ and $NH_2$], 2.29 (m, 2H, $CH_2CO_2$-), 3.50 (m, 1H, α-CH), 3.64 (s, 3H, $OCH_3$), 3.69 (s, 3H, $OCH_3$).

The hydrochloride salt is prepared in 79% yield by treating the base with anhydrous HCl in $Et_2O$. The product is crystallized from EtOAc-hexane in the form of colorless fibers: mp 103-104 °C. Analysis for C, H, N, and Cl confirms the composition.

4-Amino-4-deoxy-$N^{10}$-methylpteroic acid (0.36 g, 0.001 mol) is added to a solution of diethyl phosphorocyanidate (0.41 g, 0.0025 mol) and $Et_3N$ (0.25 g, 0.0025 mol) in dry DMF (40 ml). The mixture is stirred at room temperature overnight, and additional portions of diethyl phosphorocyanidate (0.21 g, 0.0013 mol) and $Et_3N$ (0.13 g, 0.0013 mol) are added to complete the activation of the COOH group. After 2 h, a solution of dimethyl L-α-aminosuberate (0.20 g, 0.009 mol) in a small volume of DMF is added, and the reaction mixture is left to stir at room temperature for 4 days. Rotary evaporation leaves a semisolid that dissolves readily in $CHCl_3$. The $CHCl_3$ solution is washed with dilute ammonia and evaporated, and the residue is chromatographed on silica gel with 95:5 $CHCl_3$-MeOH as the eluent. Appropriate TLC-homogeneous fractions are pooled and evaporated, and the residue is triturated with $Et_2O$ to obtain the diester, dimethyl N-(4-amino-4-deoxy-$N^{10}$-methylpteroyl)-L-α-aminosuberate, as a

yellow powder: 0.46 g (85); mp 112-118 °C; IR (KBr) ν 330, 1730 (ester C=O), 1615-1635 (amide C=O) $cm^{-1}$. Analysis for C, H, and N confirms the composition.

A solution of the diester (0.27 g, 0.0005 mol) in 1:1 EtOH-$H_2O$ (20 ml) is treated with $Ba(OH)_2 \cdot 8H_2O$ (0.32 g, 0.001 mol) and left to stir at room temperature overnight. To the reaction mixture is then added a solution of $Na_2SO_4$ (0.14 g, 0.001 mol) in a small volume of $H_2O$. After 5 min of vigorous stirring, the $BaSO_4$ is filtered off, and the filtrate is acidified with 10% AcOH and placed in the refrigerator until precipitation is complete. The solid is collected, washed with $H_2O$, and dried under high vacuum in a lyophilization apparatus to obtain N-(4-amino-4-deoxy-$N^{10}$-methylpteroyl)-L-α-aminosuberic acid, as a yellow powder: 0.24 g (87%); $R_f$ 0.8 (cellulose, 0.1 M phosphate buffer, pH 7.4); IR (KBr) ν 330, 2890, 1705, 1590-1625 $cm^{-1}$. Analysis for C, H, and N confirms the compostion of the compound of example 3. Corresponding analogues of AMT in which R of the generic structure is H can be prepared by substituting 4-amino-4-deoxy-pteroic acid for the 4-amino-4-deoxy-$N^{10}$-methylpteroic acid in the foregoing procedures.

CLAIMS

1. A compound having the following structure:

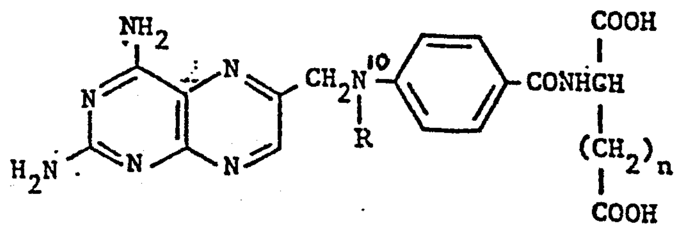

in which R is $CH_3$ or H and n is in a range of from 3 to 22.

2. A compound as claimed in claim 1 in which R is $CH_3$.

3. A compound as claimed in claim 1 in which R is H.

4. A compound as claimed in claim 2, in which n is 3, which is N-(4-amino-4-deoxy-$N^{10}$-methyl-pteroyl)-L-$\alpha$-aminoadipic acid.

5. A compound as claimed in claim 2, in which n is 4, which is N-(4-amino-4-deoxy-$N^{10}$-methyl-pteroyl)-L-$\alpha$-aminopimelic acid.

6. A compound as claimed in claim 2, in which n is 5, which is N-(4-amino-4-deoxy-$N^{10}$-methyl-pteroyl)-L-$\alpha$-aminosuberic acid.

7. A compound as claimed in claim 3, in which n is 3, which is N-(4-amino-4-deoxy-pteroyl)-L-α-aminoadipic acid.

8. A compound as claimed in claim 3, in which n is 4, which is N-(4-amino-4-deoxy-pteroyl)-L-α-aminopimelic acid.

9. A compound as claimed in claim 3, in which n is 5, which is N-(4-amino-4-deoxy-pteroyl)-L-α-aminosuberic acid.

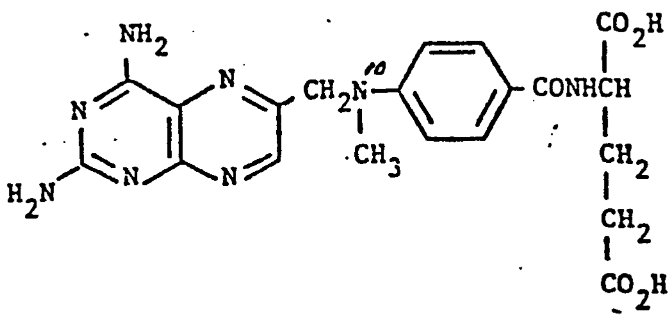

Fig. 1

0137640

European Patent Office

# EUROPEAN SEARCH REPORT

Application number

EP 84305510.4

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| X | CHEMICAL ABSTRACTS, vol. 48, no. 8, April 25, 1954, Columbus, Ohio, USA<br>V.A. KIRSANOVA et al. "The properties of amino derivatives of pteroylglutamic acid and its homologs"<br>abstract no. 4 604g<br>& Biokhimiya 18, 484-489 (1953)<br>-- | 1,3 | C 07 D 475/08<br>//A 61 K 31/505 |
| X | CHEMICAL ABSTRACTS, vol. 77, no. 23, December 4, 1972, Columbus, Ohio, USA<br>A.V. TRUFANOV et al. "Bioligical and therapeutic role of 4-amino pteroyl amino-adipic acid"<br>page 70, column 1, abstract no. 148 071j<br>& Vop. Med. Khim., 18(4), 418-21, (1972)<br>-- | 1,3 | TECHNICAL FIELDS SEARCHED (Int. Cl.4)<br>C 07 D 475/00 |
| A | CHEMICAL ABSTRACTS, vol. 85, no. 23, December 6, 1976, Columbus, Ohio, USA<br>A. ROSOWSKY et al. "Methotrexate analogs"<br>page 574, column 2, abstract no. 177 930j<br>& J. Hetero cycl. Chem. 13(4), 727-732 (1976)<br>---- | | |

The present search report has been drawn up for all claims

| Place of search | Date of completion of the search | Examiner |
|---|---|---|
| VIENNA | 15-11-1984 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document